# EUROPÄISCHE PATENTSCHRIFT

(11) **EP 1 359 958 B1**
(45) Veröffentlichungstag und Bekanntmachung des Hinweises auf die Patenterteilung: **18.04.2007**
(21) Anmeldenummer: 01911436.2
(22) Anmeldetag: 09.02.2001
(51) Int. Cl.: A61M 5/24, A61M 5/32, A61M 5/34

(54) **INJEKTIONSSPRITZE**
INJECTION SYRINGE
SERINGUE D'INJECTION

(43) Veröffentlichungstag der Anmeldung: 12.11.2003
(73) Patentinhaber: Hager & Werken Gmbh & Co. KG, 47269 Duisburg (DE)
(72) Erfinder: KRANTZ, Heiko, 47495 Rheinberg (DE); KONRAD, Peter, 42781 Haan (DE)
(74) Vertreter: Sroka, Peter-Christian
(86) Internationale Anmeldenummer: PCT/DE2001/000515
(87) Internationale Veröffentlichungsnummer: WO 2002/064197

(56) Entgegenhaltungen:
- EP-A- 0 787 501

## Beschreibung

Die Erfindung betrifft eine Injektionsspritze gemäß dem Oberbegriff des Patentanspruchs 1.

Eine den Ausgangspunkt der vorliegenden Erfindung bildende und in der EP 0 787 501 A2 beschriebene Injektionsspritze enthält im wesentlichen folgende Elemente:
- ein Gehäuse zur Aufnahme eine mit einem Dichtkörper ausgerüsteten Medikamentenampulle,
- einen in Gehäuselängsrichtung verschiebbaren Kolben zum Eindrücken des Dichtkörpers in eine Medikamentampulle;
- eine Halterung für eine Injektionsnadeleinheit, die einen lösbar in die Halterung einsetzbaren, nabenähnlichen Kanülenträger aufweist, der an seinem hinteren Ende einen radial nach außen gerichteten Flanschring enthält und in den eine nach vom und nach hinten herausragende Kanüle eingesetzt ist,
- eine Spreizhülse mit elastischen Spreizzungen, die an ihren vorderen Enden radial nach innen gerichtete, bei in die Halterung eingesetzter Injektionsnadeleinheit hinter den Flanschring des Kanülenträgers greifende Nasen sowie ebenfalls radial gerichtete Spreizvorsprünge aufweisen und mit ihren hinteren Enden an einem an dem Spitzengehäuse befestigten Zylinderring angebracht sind, und
- eine äußere gegen die Kraft einer Feder nach hinten verschiebbare Schiebehülse, die so gestaltet ist, daß bei ihrer nach hinten gerichteten Bewegung gegen die Spreizvorsprünge gedrückt wird und damit die Spreizzungen zwecks Freigabe der Injektionsnadeleinheit radial nach außen gedrückt werden.

Bei dieser bekannten Injektionsspritze weist der Spreizkörper drei Spreizzungen auf, die an ihren dem Zylinderring zugewandten hinteren Enden radial nach innen gerichtete Spreizvorsprünge haben. Die Schiebehülse ist mit radial nach außen gerichteten Vorsprüngen versehen, die beim Verschieben der Schiebehülse in Richtung des Spritzengehäuses gegen die die an den Spreizzungen angebrachten Spreizvorsprünge drükken, wodurch die Spreizzungen insgesamt nach außen gedrückt werden und dadurch die Injektionsnadeleinheit freigeben.

Die an den hinteren Enden der Spreizzungen angebrachten Spreizvorsprünge sowie die mit den nach außen gerichteten Vorsprüngen versehene Schiebehülse bedingen einen erhöhten, auch die Herstellung erschwerenden konstruktiven Aufwand, abgesehen davon, daß bei nur drei Spreizzungen nicht immer ein sicheres Einsetzen und Fixieren der Injektionsnadel-einheit in die bzw. der Halterung gewährleistet ist.

Der Erfindung liegt die Aufgabe zugrunde, eine Injektionsspritze zu schaffen, deren Halterung zwecks einfacher Herstellung in konstruktiver Hinsicht einfacher ausgestaltet ist, als es bei der bekannten Injektionsspritze der Fall ist, wobei zusätzlich auch noch ein sichereres Fixieren der Injektionsnadeleinheit in der Halterung gewährleistet sein soll.

Zur Lösung dieser Aufgabe ist die erfindungsgemäße Injektionsspritze dadurch gekennzeichnet, daß der Spreizkörper mindestens vier einen Ringkäfig bildende Spreizzungen aufweist und daß die Schiebehülse an ihrem vorderen Ende ein ringförmiges Verstellelement enthält, mit dem die Spreizzungen bei der nach hinten gerichteten Bewegung der Schiebehülse radial nach außen gedrückt werden.

Bei der Schiebehülse handelt es sich damit um einen insgesamt rotationssymmetrischen Körper, der relativ einfach in der Herstellung ist, und zwar im Gegensatz zu der bei der bekannten Spritze verwendeten Schiebehülse, die entsprechend der Anzahl der Spreizzungen mit nach außen gerichteten Vorsprüngen versehen ist. Bei der erfindungsgemäß verwendeten Halterung können auch die mit den nach außen gerichteten Vorsprüngen der Schiebehülse zusammenwirkenden Vorsprünge bzw. Verdickungen an den Spreizzungen entfallen.

Die Erfindung wird im folgenden anhand der Zeichnungen beschrieben:
Figur 1 zeigt eine Seitenansicht der erfindungsgemäßen Injektionsspritze mit darin eingesetzter Injektionsnadeleinheit;
Figur 2 zeigt einen Axialschnitt der erfindungsgemäß gestalteten, auf das Spritzengehäuse aufschraubbaren Halterung für die Injektionsnadeleinheit, welche Halterung im wesentlichen eine Spreizhülse, einen rohrförmigen Führungskörper für die Injektionsnadeleinheit, einen Nadelauswerfer sowie eine äußere Schiebehülse umfaßt;
Figur 3 zeigt im Axialschnitt die Spreizhülse;
Figur 4 zeigt eine Seitenansicht des Führungskörpers;
Figur 5 zeigt eine Schnittansicht des Nadelauswerfers;
Figur 6 zeigt ausschnittsweise eine Schnittansicht des Deckels eines Entsorgungsbehälters;
Figur 7 zeigt eine teilweise Draufsicht auf einen Deckel gemäß Figur 6.

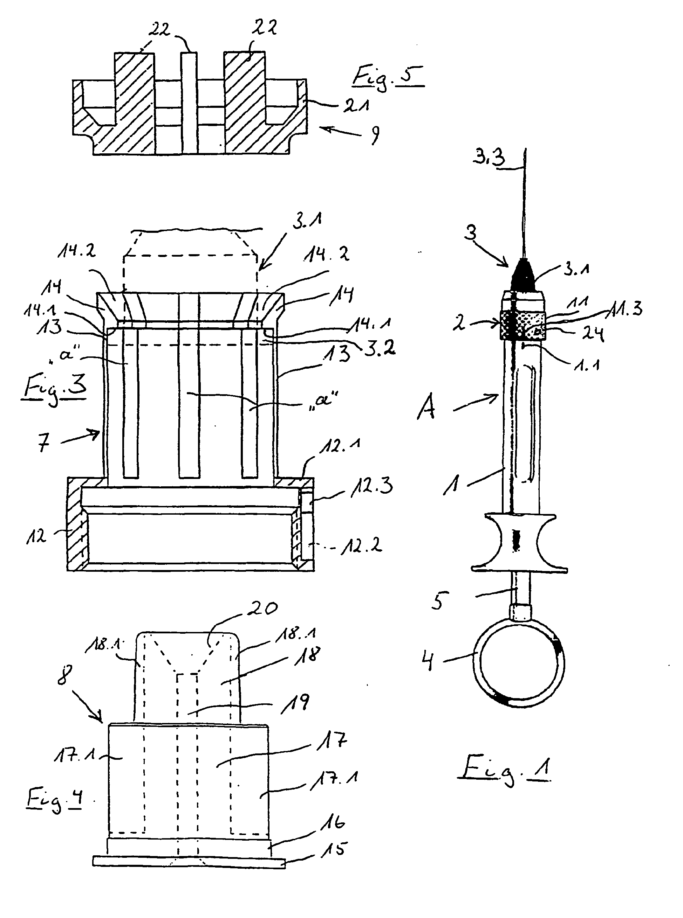

Die in Figur 1 dargestellte Injektionsspritze A hat ein Gehäuse 1 zur Aufnahme einer üblichen und daher nicht dargestellten Medikamentenampulle, die an ihrem hinteren Ende durch einen Dichtkörper und an ihrem vorderen Ende durch eine Membran verschlossen ist. In das vordere Ende der Injektionsspritze A ist mittels der im folgenden noch zu beschreibenden Halterung 2 der Kanülenträger 3 einer Injektionsnadeleinheit B lösbar eingesetzt, die einen durch die Membran einer Medikamentampulle einstechbaren hinteren Kanülenabschnitt aufweist. Eine mittels eines Handgriffs 4 in das Gehäuse 1 einschiebbare Stange 5 trägt an ihrem vorderen Ende einen (nicht dargestellten) Stößel, der beim Einschieben der Stange 5 in das Gehäuse den die Ampulle am hinteren Ende verschließenden Dichtkörper in die Ampulle eindrückt, wodurch das in der Ampulle befindliche Medikament durch den äußeren Injektionskanülenabschnitt 6 einem Patienten injiziert werden kann.

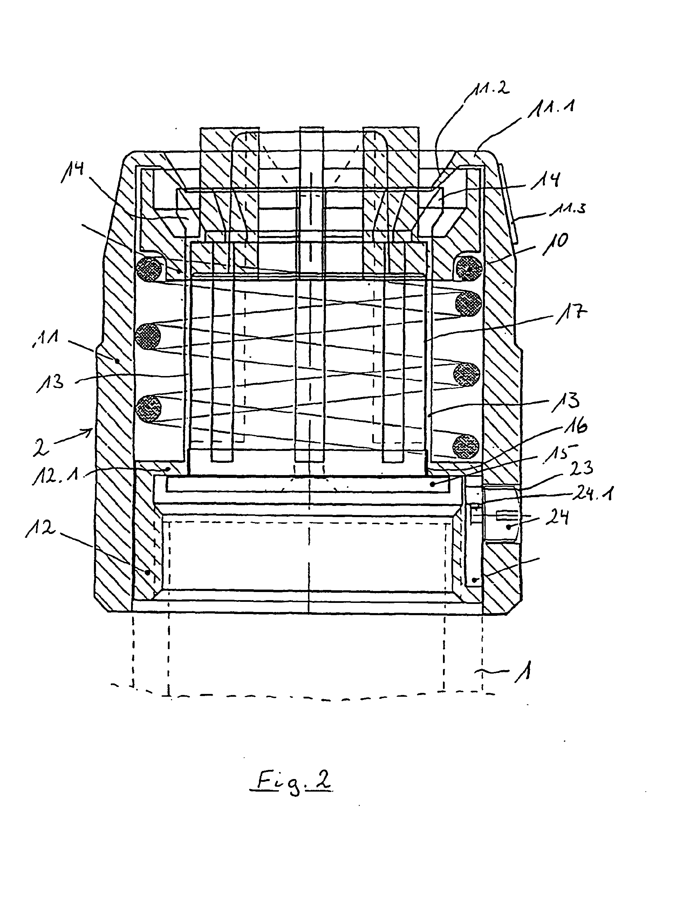

Die anhand von Figur 1 beschriebene Injektionsspritze A entspricht hinsichtlich ihres grundsätzlichen Aufbaus der in der EP 0 787 501 A2 beschriebenen Injektionsspritze. Von dieser bekannten Injektionsspritze unterscheidet sich der Anmeldungsgegenstand im wesentlichen durch die Konstruktion der in Figur 2 dargestellten Halterung 2.

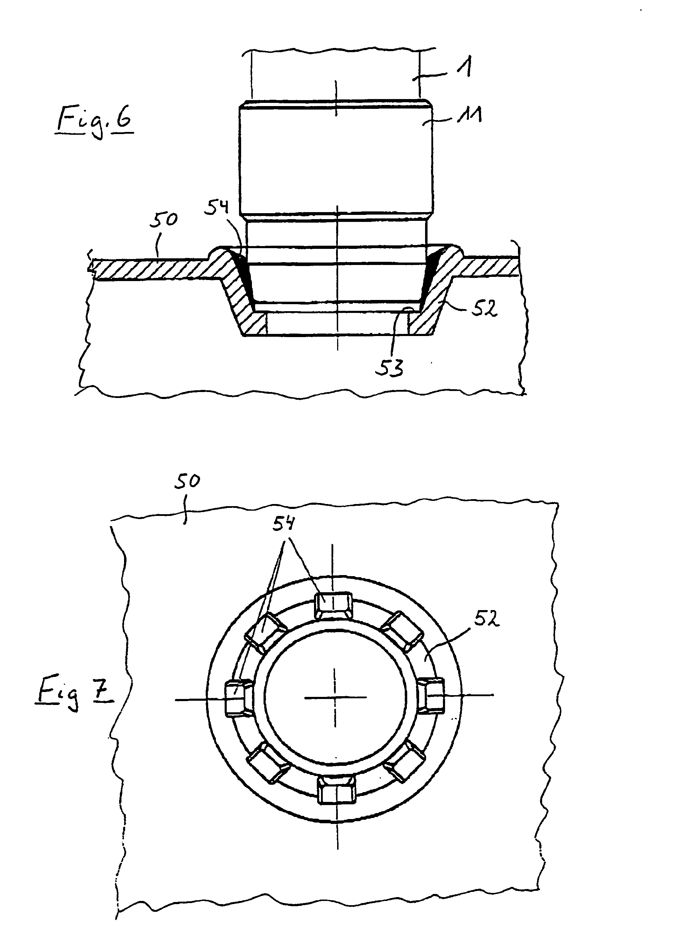

Diese Halterung 2 besteht im einzelnen aus einer als Einzelteil in Figur 3 dargestellten Spreizhülse 7, in die der gestrichelt angedeutete Kanülenansatz 3 eingesetzt ist, einem von unten in die Spreizhülse 7 einschiebbaren vorzugsweise im Preßsitz eingesetzten, rohrförmigen Führungskörper 8 (siehe Figur 4), einem in Figur 5 dargestellten Nadelauswerfer 9, der gegen die Kraft einer Feder 10 relativ zur Zentrierhülse 7 verschiebbar ist, sowie eine äußere Schiebehülse 11, die in noch zu beschreibender Weise gegenüber der auf das Gehäuse 1 aufgeschraubten Spreizhülse 7 beweglich ist.

Die Spreizhülse 7 besteht aus einem ein Innengewinde aufweisende Zylinderring 12, an dessen oberes Ende ein nach innen gerichteter Flanschring 12.1 schließt. An diesem Ringflansch 12.1 sind mehrere, vorzugsweise acht, einen Ringkäfig bildende elastische Spreizzungen 13 angebracht. An dem vorderen, freien Ende jeder Spreizzunge 13 ist ein eine radial nach innen vorspringenden Nase 14.1. bildender Halterungs- und Führungskörper 14 angebracht, der eine von oben nach unten, schräg nach innen gerichtete Führungsfläche 14.2 aufweist.

Die Führungsflächen 14.2 sämtlicher Spreizzungen 13 bilden eine durch Spalten a zwischen benachbarten Spreizzungen 13 unterbrochenen Konusfläche.

Der in Figur 4 dargestellte und von unten in die Spreizhülse 7 z. B. im Preßsitz eingesetzte Führungskörper 8 hat einen gegen die Unterseite des Flanschringes 12.1 anliegenden Flanschring 15, einen gegen den Innenrand des Flanschringes 12.1 anliegenden Paß- und Zentrierbund 16, einen mittleren Zylinderabschnitt 17 mit gegenüber dem Paßring 16 verminderten Durchmesser, sowie einem oberen Zylinderabschnitt 18. Der Führungskörper 8 ist mit einer durchgehenden Zentralbohrung 19 versehen, in die am oberen Ende des oberen Zylinderabschnitts 18 eine Konusbohrung 20 einmündet.

Die mittleren und oberen Zylinderabschnitte 17 und 18 sind mit beispielsweise vier radial nach innen gerichteten und in Achsrichtung verlaufenden Aussparungen 17.1 bzw. 18.1 versehen, die im fertigen Montagezustand der Halterung 2 den zwischen den Spreizzungen 13 befindlichen Spalten a gegenüberliegen bzw. damit fluchten.

Der Nadelauswerfer 9 besteht aus einem Ring 2.1, an dem beispielsweise vier radial nach innen gerichtete, blattförmige Auswerferflossen 2.2 angebracht sind, die über die Oberseite des Ringes 21 hinausragen. Die Anordnung der Auswerferflossen 2.2 ist derart, daß sie während der Axialverschiebung des Nadelauswerfers 9 durch die zwischen den Zylinderzungen befindlichen Spalten a hindurch in den Aussparungen 17.1 bzw. 18.1 geführt sind.

Die Schiebehülse 11 hat im wesentlichen die Form einer Zylinderbuchse, an deren oberen Rand ein nach innen gerichteter Flanschring 11.1 1 anschließt, gegen dessen Unterseite der obere Rand des Rings 21 des Nadelauswerfers 9 von der Feder 10 gedrückt wird. An diesen Flanschring 11.1 schließt ein im Querschnitt zungenförmiger, schräg nach innen und hinten gerichteter Konusring 11.2 an, dessen unterer Rand in den durch die Führungsflächen 14.2 der Spreizzungen 13 gebildeten Konusfläche ragt.

Die Gewindebuchse 12 der Spreizhülse 7 ist an ihrem Außenumfang mit einer Axialnut 12.2 versehen, an deren oberes Ende sich ein in Umfangsrichtung der Gewindebuchse erstreckender Nutabschnitt 12.3 anschließt. Die Schiebehülse 11 ist mit einer radial gerichteten Gewindebohrung 23 zum Einschrauben einer Madenschraube 24 versehen, die einen in die Nut 12.2 bzw. 12.3 ragenden Vorsprung 24.1 hat. Wenn der Vorsprung 24.1 in die Umfangsnut 12.3 ragt, ist die Schiebehülse 11 gegen Axialverschiebung gesichert. Dieser Zustand entspricht dem Gebrauchszustand der Injektionsspritze A bei in die Halterung 2 eingesetzter Injektionsnadeleinheit B. Dieser Zustand wird durch am Außenumfang der Schiebehülse 11 einerseits und am Außenumfang des Spritzengehäuses 1 angebrachte, miteinander fluchtende Markierungen 1.1 und 11.3 angezeigt. In Figur 1 zeigen die Markierungen 1.1 und 11.3 den Zustand an, bei dem der Vorsprung 24.1 der Nabenschraube 24 sich am Ende der Umfangsnut 12.3 befindet, so daß die Schiebehülse 11 gegen Axialverschiebung gesichert ist.

Zum Einsetzen einer Injektionsnadeleinheit in die Halterung 2 wird zuerst der nicht dargestellte hintere Kanülenabschnitt durch die Konusbohrung 20 in die Zentralbohrung 19 eingeschoben, bis der radial nach außen gerichtete Flanschring 3.2 des Kanülenträgers 3.1 gegen die Führungsflächen 14.2 der Spreizzungen 13 zur Anlage kommt. Durch weiteres Eindrük-ken des Kanülenträgers 3.1 in die Halterung 2 gleitet der Flanschring 3.2 des Kanülenträgers 3.1 entlang der Führungsflächen 14.2, wodurch die Spreizzungen 13 nach außen gedrückt werden, und zwar so lange, bis der Flanschring 3.2 die Halterungs- und Führungskörper 14 passiert hat, so daß die Spreizzungen 13 wieder in ihre Ausgangslage zurückfedern können und der Flanschring 3.2 eine Position unterhalb der Nasen 14.1 einnimmt, so wie es in Figur 3 dargestellt ist.

Die Unterseite des Flanschrings 3.2 wird in diesem Zustand auf der den oberen Zylinderabschnitt 1.8 des Führungskörpers 8 umgebenden Ringschulter abgestützt, die durch die freie Oberseite des mittleren Zylinderabschnitts 1.7 gebildet ist.

Die Injektionsnadeleinheit 3 ist damit gegen Axialverschiebung in beiden Richtungen gesichert, sofern die Schiebehülse 11 die in Figur 1 angedeutete Position einnimmt.

Zum Herausnehmen bzw. Herauswerfen der Injektionsnadeleinheit 3 nach deren Gebrauch wird die Schiebehülse 11 relativ zum Gehäuse 1 in Richtung der am Gehäuse 1 angebrachten Markierung 1.1 gedreht, wodurch der Vorsprung 23 der Madenschraube 24 entlang der Umgangsnut 12.3 in Richtung zur Axialnut 12.2 verstellt wird. Sobald der Madenschraubenvorsprung 23 in den Bereich der Axialnut 12.2 kommt und das System damit "entsichert" ist, kann die Schiebehülse 11 entgegen der Federkraft der zwischen dem Nadelauswerfer 9 und dem Ringflansch 12.1 der Spreizhülse 7 abgestützten Rückstellfeder 10 nach hinten verschoben werden. Dieses hat zur Folge, daß der am vorderen Ende der Schiebehülse 7 angeordnete, schräg nach innen und hinten gerichtete Konusring 11.2 gegen die an den Spreizzungen 13 angebrachten, schräg nach innen gerichteten Führungsflächen 14.2 zur Anlage kommt und damit bei weiterem Verschieben der Schiebehülse 11 nach hinten die Spreizzungen 13 nach außen drückt. Dadurch kommt der am Kanülenansatz 3.1 befindliche Flanschring 3.2 von den Halterungs- und Führungskörpern 14 bzw. deren Nasen 14.1 frei, so daß der Kanülenansatz 3.1 und damit die gesamte Injektionsnadeleinheit 3 unter dem Einfluß der auf den Nadelauswerfer 9 einwirkenden Rückstellfeder 10 aus der Halterung 2 ausgeworfen wird.

Dieses Auswerfen erfolgt vorzugsweise in einen Entsorgungsbehälter, dessen in den Figuren 6 und 7 angedeuteter Deckel 50 eine Kreisöffnung aufweist, an die ein sich konisch nach unten verjüngender Rohrabschnitt 52 anschließt, an dessen unterer Rand ein nach innen gerichteter Ringflansch 53 anschließt, und daß an der Innenwand des Rohrabschnitts 52 mindestens ein Halteelement 54 angebracht ist, das in der Lage ist, bei einer von oben in diesen Rohrabschnitt 52 eingesteckten Schiebhülse 11 einer Injektionsspritze die Schiebehülse 11 gegen Drehung relativ zu dem Entsorgungsbehälter und damit auch zur Injektionsspritze festzuhalten.

Gemäß Figur 7 sind an der Innenwand des Rohrabschnitts 52 vorzugsweise acht Halteelemente in Form von entlang von Mantellinien des Rohrabschnitts 52 verlaufenden Klemmstegen 54 angebracht.

Das vordere Ende der Schiebehülse 11 hat einen sich konisch zum vorderen Schiebehülsenende verjüngenden Abschnitt, an dessen Außenseite mindestens eine zwischen die Klemmstege 54 einschiebbare Rippe 11.3 angebracht ist.

Zum beriihrungsfreien Auswerfen einer gebrauchten Injektionsnadeleinheit 3 wird die Injektionsspritze mit ihrer Schiebehülse 11 in den Rohrabschnitt 52 des ansonsten nicht dargestellten Entsorgungsbehälters eingesetzt, wodurch die Schiebehülse 11 innerhalb des Rohrabschnitts 52 gegen Drehung gesichert ist.

Durch Drehen des Gehäuses 1 wird das System anschließend in der oben beschriebenen Weise "entsichert", so daß die Injektionsspritze mit ihrer Spreizhülse 7 in Richtung des Entsorgungsbehälters verschoben werden kann, wodurch die Spreizzungen 13 in der zuvor beschriebenen Weise von dem Konusring 11.2 nach außen gedrückt werden und die Injektionsnadeleinheit 3 bzw. deren Kanülenansatz 3.1 freigeben.

## Patentansprüche

1. Injektionsspritze, enthaltend
- ein Gehäuse (1) zur Aufnahme einer mit einem Dichtkörper ausgerüsteten Medikamentenampulle,
- einen in Gehäuselängsrichtung verschiebbaren Kolben zum Eindrücken des Dichtkörpers in eine Medikamentampulle;
- eine Halterung (2) für eine Injektionsnadeleinheit (3), die einen lösbar in die Halterung (2) einsetzbaren, nabenähnlichen Kanülenträger (3.1) aufweist, der an seinem hinteren Ende einen radial nach außen gerichteten Flanschring (3.2) enthält und in den eine nach vom (3.3) und nach hinten herausragende Kanüle eingesetzt ist,
- eine Spreizhülse (7) mit elastischen Spreizzungen (13), die an ihren vorderen Enden radial nach innen gerichtete, bei in die Halterung eingesetzter Injektionsnadeleinheit hinter den Flanschring (3.2) greifende Nasen (14.1) sowie ebenfalls radial gerichtete Spreizvorsprünge aufweisen und mit ihren hinteren Enden an einem an dem Spritzengehäuse (1) befestigten Zylinderring (12) angebracht sind, und
- eine äußere gegen die Kraft einer Feder (10) nach hinten verschiebbare Schiebehülse (11), die so gestaltet ist, daß sie bei ihrer nach hinten gerichteten Bewegung gegen die Spreizvorsprünge gedrückt wird und damit die Spreizzungen zwecks Freigabe der Injektionsnadeleinheit radial nach außen gedrückt werden,
**dadurch gekennzeichnet, daß** die Spreizhülse (7) mindestens vier einen Ringkäfig bildende Spreizzungen (13) und die Schiebehülse (11) an ihrem vorderen Ende ein ringförmiges Verstellelement (11.2) enthält, mit dem die Spreizzungen (13) bei der nach hinten gerichteten Bewegung der Schiebehülse (11) radial nach außen gedrückt werden.

2. Injektionsspritze nach Anspruch 1, **dadurch gekennzeichnet, daß** die Spreizzungen (13) an den Innenseiten ihrer vorderen, freien Enden schräg von oben nach unten, radial nach innen gerichtete, die Spreizvorsprünge bildende Führungsflächen (14.2) haben.

3. Injektionsspritze nach Anspruch 1 oder 2, **dadurch gekennzeichnet, daß** das ringförmige Ventellelement (11.2) einen schräg nach innen und hinten gerichteten, im Querschnitt zungenförmigen Konusring (11.2) zum Zusammenwirken mit den Spreizvorsprüngen der Spreizzungen (13) aufweist.

4. Injektionsspritze nach Anspruch 1, **dadurch gekennzeichnet, daß** die Spreizhülse (7) acht Spreizzungen (13) hat.

5. Injektionsspritze nach einem der Ansprüche 1 bis 4, **gekennzeichnet durch** einen innerhalb der Schiebehülse (11) geführten, federbelasteten Nadelauswerfer (9), enthaltend einen die Spreizzungen (13) umgebenden und relativ dazu verschiebbaren Ring (21), an dessen Innenseite radial nach innen gerichtete, zwischen die Spreizzungen (13) greifende Auswerferflossen (22) angebracht sind, die über die Oberseite des Ringes (21) ragen.

6. Injektionsspritze nach Anspruch 5, **dadurch gekennzeichnet, daß** die Feder (10) zwischen der Unterseite des Auswerferringes (9) und einem Flanschring (12.1) der Spreizhülse (7) abgestützt ist, welcher Ringflansch an den Zylinderring (12) anschließt und an seinem inneren Rand die Spreizzungen (13) trägt.

7. Injektionsspritze nach einem der Ansprüche 1 bis 6, **gekennzeichnet durch** einen zentralen, rohrförmigen Führungskörper (8) zur Führung des inneren in die Ampulle einstechbaren Kanülenabschnitts der Injektionsnadeleinheit (3), welcher Führungskörper (8) fest mit der Spreizhülse (7) verbunden ist.

8. Injektionsspritze nach Anspruch 1, **dadurch gekennzeichnet, daß** in der Schiebehülse (11) eine Gewindebohrung (23) zum Einschrauben einer Schraube (24) in eine am Außenumfang die Spreizhülse (7) angebrachte Axialnut (12.2) angeordnet ist, deren Länge den Verschiebeweg der Schiebehülse (11) bestimmt.

9. Injektionsspritze nach Anspruch 8, **dadurch gekennzeichnet, daß** sich an das vordere Ende der Axialnut (12.2) ein in Umfangsrichtung der Spreizhülse (7) verlaufender Nutabschnitt (12:3) anschließt, der eine Drehbewegung der Schiebehülse (11) relativ zur Spreizhülse (7) ermöglicht.

10. Injektionsspritze nach Anspruch 9, **gekennzeichnet durch** am Außenumfang der Schiebehülse (11) und am Außenumfang des Spritzengehäuses (1) angeordnete Markierungen (11.3 bzw. 1.1), um die Drehposition der Schiebehülse (7) relativ zum Spritzengehäuse (1) anzuzeigen, in der eine Axialverschiebung der Schiebehülse (7) nicht möglich ist.

11. Injektionsspritze nach einem der Ansprüche 1 bis 10, **dadurch gekennzeichnet, daß** das vordere Ende der Schiebehülse (11) einen sich konisch zum vorderen Schiebehülsenende verjüngenden Abschnitt hat, an dessen Außenseite mindestens eine gegen einen Klemmsteg (54) zur Anlage bringbare Rippe (11.3) angebracht ist.

## Claims

1. Injection syringe containing:
- a casing (1) for receiving an ampoule of medicine equipped with a sealing member;
- a piston, which is displaceable in the longitudinal direction of the casing, for pressing said sealing member into an ampoule of medicine;
- an arrangement (2) for holding an injection-needle unit (3) which has a hub-like cannula-carrier (3.1) which can be inserted in said holding arrangement (2) in a detachable manner and contains, at its rear end, a flanged ring (3.2) which is directed radially outwards, and in which cannula-carrier a cannula which projects forwards (3.3) and rearwards is inserted;
- a spreading sleeve (7) with elastic spreading tongues (13) which have, at their front ends, noses (14.1) which are directed radially inwards and engage behind the flanged ring (3.2) when the injection-needle unit is inserted in the holding arrangement, and which elastic spreading tongues likewise have radially directed spreading projections and are attached, by their rear ends, to a cylindrical ring (12) fastened to the casing (1) of the syringe; and
- an outer sliding sleeve (11) which is displaceable rearwards against the force of a spring (10) and which is configured in such a way that, in the course of its rearwardly directed movement, is pressed against the spreading projections and the spreading tongues are thereby pressed radially outwards for the purpose of releasing the injection-needle unit;
**characterised in that** the spreading sleeve (7) contains at least four spreading tongues (13) which form an annular cage and the sliding sleeve (11) contains, at its front end, an annular adjusting element (11.2) by means of which said spreading tongues (13) are pressed radially outwards in the course of the rearwardly directed movement of said sliding sleeve (11).

2. Injection syringe according to Claim 1, **characterised in that** the spreading tongues (13) have, on the inner sides of their front, free ends, guide faces (14.2) which are directed obliquely from top to bottom and radially inwards and which form the spreading projections.

3. Injection syringe according to Claim 1 or 2, **characterised in that** the annular adjusting element (11.2) has a cone ring (11.2), which is directed obliquely inwards and rearwards and is tongue-shaped in cross-section, for interacting with the spreading projections on the spreading tongues (13).

4. Injection syringe according to Claim 1, **characterised in that** the spreading sleeve (7) has eight spreading tongues (13).

5. Injection syringe according to one of Claims 1 to 4, **characterised by** a spring-loaded needle-ejector (9) which is guided inside the sliding sleeve (11) and contains a ring (21) which surrounds the spreading tongues (13) and is displaceable relative to the latter and on the inner side of which are located ejector fins (22) which are directed radially inwards and engage between the spreading tongues (13) and which project above the upper side of said ring (21).

6. Injection syringe according to Claim 5, **characterised in that** the spring (10) is supported between the underside of the ejector ring (9) and a flanged ring (12.1) on the spreading sleeve (7), which annular flange adjoins the cylindrical ring (12) and carries the spreading tongues (13) on its inner edge.

7. Injection syringe according to one of Claims 1 to 6, **characterised by** a central, tubular guide member (8) for guiding that inner, cannula portion of the injection-needle unit (3) which can be stuck into the ampoule, which guide member (8) is fixedly connected to the spreading sleeve (7).

8. Injection syringe according to Claim 1, **characterised in that** there is disposed, in the sliding sleeve (11), a threaded bore (23) for screwing a screw (24) into an axial groove (12.2) which is located on the outer periphery of the spreading sleeve (7) and the length of which determines the path of displacement of the sliding sleeve (11).

9. Injection syringe according to Claim 8, **characterised in that** the front end of the axial groove (12.2) is adjoined by a portion (12.3) of the groove which extends in the peripheral direction of the spreading sleeve (7) and which permits a rotational movement of the sliding sleeve (11) relative to said spreading sleeve (7).

10. Injection syringe according to Claim 9, **characterised by** markings (11.3 and 1.1) which are disposed, respectively, on the outer periphery of the sliding sleeve (11) and on the outer periphery of the casing (1) of the syringe, in order to indicate that rotational position of said sliding sleeve (7), relative to the casing (1) of the syringe, in which axial displacement of said sliding sleeve (7) is not possible.

11. Injection syringe according to one of Claims 1 to 10, **characterised in that** the front end of the sliding sleeve (11) has a portion which tapers conically towards the front end of said sliding sleeve and on the outer side of which there is located at least one rib (11.3) which can be brought into abutment against a gripping web (54).

## Revendications

1. Seringue à injection, contenant
- un logement (1) en vue de recevoir une ampoule médicamenteuse équipée d'un corps d'étanchéité,
- un piston pouvant être déplacé dans la direction longitudinale du logement en vue d'enfoncer le corps d'étanchéité dans une ampoule médicamenteuse,
- un support (2) pour une unité d'aiguille d'injection (3) qui présente un porte-canule (3.1) semblable à un moyeu, pouvant être inséré de manière amovible dans le support (2) qui contient au niveau de son extrémité arrière un collier (3.2) orienté radialement vers l'extérieur et dans lequel une canule dépassant vers l'avant (3.3) et vers l'arrière est insérée,
- un manchon d'écartement (7) avec des languettes d'écartement élastiques (13) qui présentent des nez (14.1) orientés radialement vers l'intérieur, s'accrochant derrière le collier (3.2) lorsque l'unité d'aiguille d'injection est insérée dans le support ainsi que des saillies d'écartement également orientées radialement et sont appliquées avec leurs extrémités arrière sur un anneau cylindrique (12) fixé au logement de la seringue (1), et
- un manchon coulissant extérieur (11) pouvant être déplacé vers l'arrière à l'encontre de la force d'un ressort (10) et qui est configuré de sorte qu'en cas de son mouvement orienté vers l'arrière, une poussée est effectuée contre les saillies d'écartement et les languettes d'écartement sont ainsi poussées radialement vers l'extérieur dans le but de libérer l'unité d'aiguille d'injection,
**caractérisée en ce que** le manchon d'écartement (7) contient au moins quatre languettes d'écartement (13) formant une cage annulaire et le manchon coulissant (11) contient au niveau de son extrémité avant un élément de déplacement annulaire (11.2) avec lequel les languettes d'écartement (11) sont poussées radialement vers l'extérieur lors du mouvement orienté vers l'arrière du manchon coulissant (11).

2. Seringue à injection selon la revendication 1,
**caractérisée en ce que** les languettes d'écartement (13) ont des surfaces de guidage (14.2) sur les côtés internes de leurs extrémités avant, libres orientées en biais du haut vers le bas, radialement vers l'intérieur, formant les saillies d'écartement.

3. Seringue à injection selon la revendication 1 ou 2,
**caractérisée en ce que** l'élément de déplacement annulaire (11.2) présente un anneau conique (11.2) en forme de langue en section transversale, orienté en biais vers l'intérieur et l'arrière en vue de coopérer avec les saillies d'écartement des languettes d'écartement (13).

4. Seringue à injection selon la revendication 1,
**caractérisée en ce que** le manchon d'écartement (7) a huit languettes d'écartement (13).

5. Seringue à injection selon l'une quelconque des revendications 1 à 4, **caractérisée par** un éjecteur d'aiguille (9) sollicité par un ressort, guidé au sein du manchon coulissant (11), contenant un anneau (21) entourant les languettes d'écartement (13) et pouvant être déplacé par rapport à elles, sur le côté interne duquel des stabilisateurs d'éjecteur (22) orientés radialement vers l'intérieur, s'accrochant entre les languettes d'écartement (13) sont appliqués, lesquels dépassent au-dessus du côté supérieur de l'anneau (21).

6. Seringue à injection selon la revendication 5,
**caractérisée en ce que** le ressort (10) est appuyé entre le côté inférieur de l'anneau éjecteur (9) et un collier (12.1) du manchon d'écartement (7), lequel collier se raccorde à l'anneau cylindrique (12) et porte sur son bord interne les languettes d'écartement (13).

7. Seringue à injection selon l'une quelconque des revendications 1 à 6, **caractérisée par** un corps de guidage central tubulaire (8) en vue du guidage de la portion de canule interne pouvant être enfoncée dans l'ampoule de l'unité d'aiguille d'injection (3), lequel corps de guidage (8) est relié fixement au manchon d'écartement (7).

8. Seringue à injection selon la revendication 1,
**caractérisée en ce qu'**un alésage fileté (23) est disposé dans le manchon coulissant (11) en vue de visser une vis (24) dans une rainure axiale (12.2) appliquée à la périphérie externe du manchon d'écartement (7) dont la longueur détermine la course de déplacement du manchon coulissant (11).

9. Seringue à injection selon la revendication 8,
**caractérisée en ce qu'**une portion de rainure (12.3) s'étendant dans la direction périphérique du manchon d'écartement (7) qui permet un mouvement de rotation du manchon coulissant (11) par rapport au manchon d'écartement (7), se raccorde à l'extrémité avant de la rainure axiale (12.2).

10. Seringue à injection selon la revendication 9,
**caractérisée par** des marquages (11.3 ou 1.1) disposés à la périphérie externe du manchon coulissant (11)et à la périphérie externe du logement de la seringue (1) afin d'afficher la position de rotation du manchon coulissant (7) par rapport au logement de la seringue (1), dans lequel un déplacement axial du manchon coulissant (7) n'est pas possible.

11. Seringue à injection selon l'une quelconque des revendications 1 à 10, **caractérisée en ce que** l'extrémité avant du manchon coulissant (11) a une portion se rétrécissant de manière conique vers l'extrémité avant du manchon coulissant, sur le côté externe de laquelle au moins une nervure (11.3) pouvant être amenée en appui contre une traverse de serrage (54) est appliquée.
